(19) Europäisches Patentamt
European Patent Office
Office européen des brevets

(11) Veröffentlichungsnummer: **0 470 295 A1**

(12) # EUROPÄISCHE PATENTANMELDUNG

(21) Anmeldenummer: **90124139.8**

(22) Anmeldetag: **13.12.90**

(51) Int. Cl.5: **C07C 69/74**, C07C 69/63, C07C 67/30, C07C 233/58

(30) Priorität: **10.08.90 DE 4025403**

(43) Veröffentlichungstag der Anmeldung: **12.02.92 Patentblatt 92/07**

(84) Benannte Vertragsstaaten: **AT BE CH DE FR GB IT LI NL SE**

(71) Anmelder: **HÜLS AKTIENGESELLSCHAFT
- RSP Patente / PB 15 - Postfach 13 20
W-4370 Marl 1(DE)**

(72) Erfinder: **Kaufhold, Manfred, Dr.
Jasminweg 20
W-4370 Marl(DE)**

(54) **Verfahren zur Herstellung von 2,2-Dimethylcyclopropancarbonsäurebutylestern und deren entsprechendem Amid.**

(57) Die Erfindung betrifft ein Verfahren zur Herstellung von 2,2-Dimethylcyclopropancarbonsäurebutylester durch Umsetzung von 5,5-Dimethylbutyrolacton mit Alkohol und gasförmigem Chlorwasserstoff zum 4-Chlor-4-methylpentansäurebutylester und dessen Cyclisierung mit einem Alkalialkoholat zum 2,2-Dimethylcyclopropancarbonsäurebutylester, welches dadurch gekennzeichnet ist, daß man als Alkoholkomponente Butanole in beiden Stufen verwendet und zunächst in einer ersten Stufe 5,5-Dimethylbutyrolacton und Butanol im Molverhältnis 1 : 1 bis 1 : 5 und Chlorwasserstoff in Abwesenheit eines Katalysators bei einer Temperatur von 40 bis 90 °C umsetzt, über eine Kolonne Butanol und Wasser abdestilliert und ohne Reinigung den rohen 4-Chlor-4-methylpentansäurebutylester in die 2. Stufe einsetzt, bei der dieser Ester mit einer Lösung eines Natriumbutylats in dem entsprechenden Butanol bei 80 bis 160 °C und einem Molverhältnis von Alkoholat zur Chlorverbindung von 1 : 1 bis 3 : 1 cyclisiert.

EP 0 470 295 A1

Rank Xerox (UK) Business Services

Die Erfindung betrifft ein Verfahren zur Herstellung von 2,2-Dimethylcyclopropancarbonsäurebutylestern der Formel 1 und dem entsprechenden Amid (2) durch Umsetzung von 5,5-Dimethylbutyrolacton (3) mit Chlorwasserstoff und einem Butylalkohol zu einem 4-Chlor-4-methylpentansäureester der Formel 4 und anschließende Cyclisierung mit einem Alkoholat zu 1 sowie Umsetzung mit Ammoniak zum Amid 2.

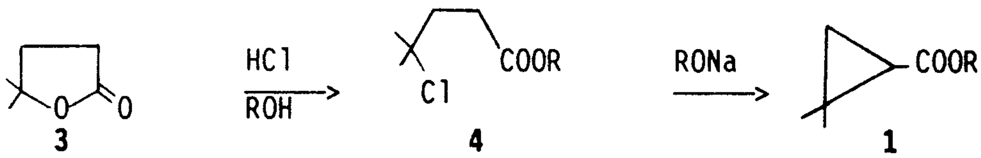


3 4 1

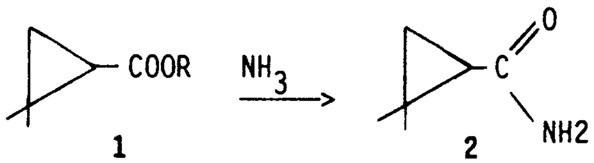


1 2

Synthesen von 4-Chlor-4-methylpentansäureestern und 2,2-Dimethylcyclopropancarbonsäureestern, die vom 5,5-Dimethylbutyrolacton ausgehen, das durch Umsetzung von Acrylsäure mit Isopropanol leicht zugänglich ist, sind literaturbekannt.

So beschreiben U. und S. Julia und Cl. Jeanmart bereits 1961 eine Synthese von 4-Chlor-4-methylpentansäureethylester und 2,2-Dimethylcyclopropancarbonsäureethylester ausgehend vom 5,5-Dimethylbutyrolacton (Bull. Soc. Chim. France 1961, 1857-60), bei der 3 bei Raumtemperatur 72 Stunden lang zur Reaktion gebracht wird. Die Aufarbeitung erfolgt mit Eiswasser-Wäsche, Extraktion und Destillation. Die 2. Stufe, die Ringbildung wird mit Natriumamylat in Benzol innerhalb von 4 Stunden erreicht. Zur Aufarbeitung ist ähnlich wie in der 1. Stufe eine Behandlung mit Eiswasser, eine Extraktion mit Ether, eine Wäsche des Ethers und eine Destillation erforderlich.

Diese Aufarbeitungen sind aufwendig und verursachen viel Abfallprodukte. Die Verwendung von festem Natriumamylat in Benzol erfordert zusätzliche Kosten bei der Herstellung dieses Gemisches und ihrer Aufarbeitung nach der Reaktion.

Dagegen wurde von R. Lantzsch (Synthesis 1982, 11, 955-956) ein Cyclisierungsverfahren vorgeschlagen, das mit der leicht handhabbaren wäßrigen Kalilauge arbeitet. Nachteilig bei dieser Arbeitsweise ist aber die zu hohe Verdünnung durch das erforderliche Lösungsmittel und die Verwendung von sehr groben Mengen an Phasentransfer-Katalysatoren - und zwar gewichtsmäßig mehr als ein Viertel des Gewichts des Einsatzprodukts (also die zu 4 analoge Verbindung). Dadurch entstehen Abwasserprobleme und hohe Einsatzstoffkosten, welche die Vorteile der Kalilauge weit übertreffen.

Alle bekannten Verfahren benötigen teure Chemikalien, sind technisch sehr aufwendig und zeitraubend und führen zu Problemen der Abfallbeseitigung. Wünschenswert ist daher ein Verfahren, bei dem 5,5-Dimethylbutyrolacton im Eintopfverfahren mittels Chlorwasserstoff und einem Alkohol in einen 4-Chlor-4-methylpentansäureester übergeführt und dieser drucklos in üblichen Reaktoren cyclisiert wird. Der entstandene Dimethylcyclopropancarbonsäureester kann beispielsweise katalytisch mit Ammoniak zum Amid umgesetzt werden.

Es besteht ein großes Interesse an einem derartigen Verfahren, nach dem man bei geringem technischen Aufwand und ohne den Einsatz von teuren Reagenzien aus 5,5-Dimethylbutyrolacton 2,2-Dimethylcyclopropancarbonsäurebutylester und aus diesem gegebenenfalls 2,2-Dimethylcyclopropancarbonsäureamid herstellen kann, weil diese Produkte wichtige Rohstoffe für Pharma-Produkte sind.

Die sich hieraus ergebende Aufgabe wird erfindungsgemäß entsprechend den Angaben der Patentansprüche gelöst.

Gegenstand der Erfindung ist daher ein Verfahren zur Herstellung von 2,2-Dimethylcyclopropancarbonsäurebutylester durch Umsetzung von 5,5-Dimethylbutyrolacton mit Alkohol und gasförmigem Chlorwasserstoff zum 4-Chlor-4-methylpentansäurebutylester und dessen Cyclisierung mit einem Alkalialkoholat zum 2,2-Dimethylcyclopropancarbonsäurebutylester, welches dadurch gekennzeichnet ist, daß man als Alkoholkomponente Butanole in beiden Stufen verwendet und zunächst in einer ersten Stufe 5,5-Dimethylbutyrolacton und Butanol im Molverhältnis 1 : 1 bis 1 : 5 und Chlorwasserstoff in Abwesenheit eines Katalysators bei einer Temperatur von 40 bis 90 °C umsetzt, über eine Kolonne Butanol und Wasser abdestilliert und ohne Reinigung den rohen 4-Chlor-4-methylpentansäurebutylester in die 2. Stufe einsetzt, bei der dieser Ester mit einer Lösung eines Natriumbutylats in dem entsprechenden Butanol bei 80 bis 160 °C und einem

Molverhältnis von Alkoholat zur Chlorverbindung von 1 : 1 bis 3 : 1 cyclisiert.

Überraschenderweise ist die erste Reaktionsstufe, die Umsetzung des 5,5-Dimethylbutyrolactons mit Chlorwasserstoff und Butanol, in sehr kurzer Zeit auf einfachste Weise durchführbar, wenn man nicht bei Raumtemperatur, sondern bei etwas erhöhter Temperatur um 60 °C arbeitet und anschließend das überschüssige Butanol bei niedrigen Temperaturen abdestilliert. Geeignete Butanole sind n-Butanol und Isobutanol. Die Umsetzung erfolgt bei 40 bis 90 °C, vorzugsweise bei 50 bis 70 °C, in Abwesenheit eines Katalysators.

Die Cyclisierung des 4-Chlor-4-methylpentansäureisobutylesters in der zweiten Reaktionsstufe gelingt überraschend glatt bei Normaldruck und mit guten Ausbeuten von 75 %, wenn man bei relativ hohen Temperaturen die Chlorverbindung zu einer Lösung eines Natriumalkoholats in dem entsprechenden Alkohol, der 4 oder mehr C-Atome hat, gibt. Hierbei werden ungewöhnlich kurze Reaktionszeiten von z. B. 1 Stunde erreicht. Längere Reaktionszeiten verringern die Ausbeuten.

Dieser Befund, daß hohe Temperaturen und kurze Zeiten günstig sind, steht im Gegensatz zu den obengenannten Literaturstellen, die Cyclisierungen bei relativ tiefen Temperaturen durchführen und dann längere Reaktionszeiten benötigen. So wählte R. Lantzsch Reaktionstemperaturen von 35 bis 40 °C und benötigt für die Umsetzung 3 Stunden. Ausdrücklich weist er auf die Gefahr der Eliminierung von Chlorwasserstoff als Nebenreaktion hin, vor allem beim Einsatz von starken Basen und bei den vorliegenden Strukturen, bei der es sich um eine tertiäre Chlorverbindung handelt, die bekanntlich besonders instabil ist im Vergleich zu sekundären und primären Verbindungen. Darum ist der Befund neu und überraschend.

Als Alkoholate bzw. Alkohole werden Butanole, insbesondere n-Butanol und Isobutanol eingesetzt. Alkohole mit 5 bis 20 C-Atomen bzw. die entsprechenden Alkoholate können zwar auch verwendet werden, bieten aber nicht die Vorteile gemäß der Erfindung. Die Verwendung einer Natrium-n- oder -iso-butylatlösung im entsprechenden Butanol ist besonders kostengünstig, weil sie aus Natronlauge und n-Butanol oder mittels Natriummethylat einfach hergestellt werden kann, ohne daß ein luftempfindlicher Feststoff verarbeitet werden muß.

Ferner wurde überraschenderweise gefunden, daß der eingesetzte 4-Chlor-4-methylpentansäurebutylester nicht durch Destillation gereinigt werden muß, sondern als Rohprodukt verwendet werden kann. Das ist wegen seiner geringen thermischen Stabilität ein großer Vorteil hinsichtlich Ausbeute und Produktionskosten.

Die praktische Durchführung der einzelnen Stufen erfolgt beispielsweise in folgender Weise:
Bei der Herstellung des 4-Chlor-4-methylpentansäurebutylesters wird ohne Katalysator gearbeitet. 5,5-Dimethylbutyrolacton und n- oder iso-Butanol wird im Molverhältnis 1 : 1 bis 1 : 5, vorzugsweise 1 : 2 bis 1 : 3, vorgelegt und bei Raumtemperatur Chlorwasserstoff eingeleitet, wobei sich das Gemisch erwärmt. Es wird so schnell Chlorwasserstoff eingeleitet, wie das Gas aufgenommen wird, bis zur Sättigung. Es wird eine Reaktionstemperatur von 40 bis 90 °C, vorzugsweise von 50 bis 70 °C, eingestellt. Wenn die Gasaufnahme beendet ist, wird die Hauptmenge an Butanol abdestilliert und der Destillationsrückstand ohne Reinigung weiterverarbeitet.

Die Cyclisierung des 4-Chlor-4-methylpentansäurebutylesters erfolgt bei 80 bis 160 °C, vorzugsweise bei 140 bis 110 °C, indem man beispielsweise eine Lösung von Natriumisobutylat in Isobutanol vorlegt, den 4-Chlor-4-methylpentansäurebutylester zugibt und das Gemisch am Rückfluß zum Sieden erhitzt. Hierbei geht die Siedetemperatur - je nach Alkoholat-Konzentration - auf tiefere Temperaturen, z. B. von 136 auf 114 °C, herunter. Die Reaktionszeit muß so kurz wie möglich gewählt werden, weil sonst vermehrt Nebenprodukte entstehen und zwar mit folgenden Strukturen:

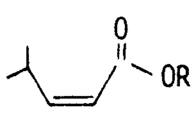
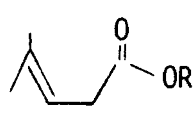
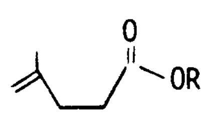

und
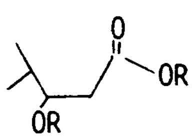

R = n- oder iso-Butyl

Das Molverhältnis Alkoholat zum 4-Chlor-4-methylpentansäurebutylester beträgt 1 : 1 bis 3 : 1,

vorzugsweise 1,8 : 1 bis 1,4 : 1.

Den erhaltenen 2,2-Dimethylcyclopropancarbonsäurebutylester setzt man beispielsweise in an sich bekannter Weise mit Ammoniak zum Amid um, das ein wichtiges Zwischenprodukt zur Herstellung von Pharma-Produkten ist. Zur Amidherstellung verwendet man ebenfalls eine Lösung von Natrium-n- oder -isobutylat im entsprechenden Butanol als Katalysator, Butanol als Lösungsmittel und einen Ammoniakdruck von beispielsweise 12 bar bei Reaktionstemperaturen von z. B. 120 °C. Diese Ausführungsform ist vorteilhaft, weil nach der Reaktion beim Abkühlen das Amid kristallin ausfällt und auf einfachste Weise durch Filtration in reiner Form gewonnen wird. Die Mutterlauge ist wieder einsetzbar.

Die folgenden Beispiele sollen das erfindungsgemäße Verfahren näher erläutern.

Beispiel 1a **4-Chlor-4-methylpentansäureisobutylester (1. Stufe)**

Man benutzt eine Glasapparatur, die aus einem Vierhalskolben mit Gaseinleitungsrohr, Rührer, Thermometer, Tropftrichter und Rückflußkühler besteht.

Man setzt sein: 798 g (7 Mol) 5,5-Dimethylbutyrolacton (99,0 %ig)
1 332 g (18 Mol) Isobutanol

Die Einsatzprodukte werden vorgelegt und sofort Chlorwasserstoff eingeleitet. Innerhalb von 4 Stunden erwärmt sich das Gemisch von 20 auf 60 °C und wird bei dieser Temperatur durch geregelte Wärmezufuhr gehalten. Es wird soviel Chlorwasserstoff eingeleitet, wie von der Lösung aufgenommen wird, bis zur völligen Sättigung. Nach 8 Stunden ist die Reaktion beendet. Die GC-Analyse zeigt folgende Zusammensetzung an:

| | |
|---|---|
| Isobutanol | 38,3 % |
| 5,5-Dimethylbutyrolacton | 2,7 % |
| 4-Chlor-4-methylpentansäureisobutylester (= CMBP) | 57,1 % |

Im Vakuum bei 124 bis 110 mbar wird die Hauptmenge an Isobutanol, 984 g, abdestilliert. Der Destillationsrückstand enthält 83,7 % CMBP, das entspricht einer Ausbeute von ca. 85 % der Theorie, bezogen auf Einsatz. Ohne Reinigung wird dieses Produkt weiterverarbeitet.

Beispiel 1 b **2,2-Dimethylcyclopropancarbonsäureisobutylester (= DCPB) (2. Stufe)**

Man benutzt eine Glasapparatur, die aus einem Dreihalskolben mit Rührer, Thermometer, Tropftrichter und 0,5 m langer, mit Glasraschigringen gefüllter Glaskolonne mit Destillationsaufsatz besteht.

Man setzt ein: 1 080 g (6 Mol) Natriummethylatlösung (30 %ig)
1 500 g (20,2 Mol) Isobutanol
1 000 g (3,87 Mol) CMBP (80 %ig)

Isobutanol und Natriummethylat werden vorgelegt und durch Abdestillieren des Methanols und wenig Isobutanol, 1 062 g, eine Lösung des Natriumisobutylats in Isobutanol hergestellt. Dabei wird eine Sumpftemperatur von 136 °C erreicht. Bei dieser Temperatur wird innerhalb einer halben Stunden CMPB zugetropft und danach noch 1 Stunde am Rückfluß zum Sieden erhitzt. Dann wird abgekühlt und das gebildete Natriumchlorid mit 1 000 g Wasser, dem 49 g Phosphorsäure zugesetzt wurden, gelöst.

Die Phasen werden getrennt, die wäßrige Phase, 1 201 g, wird verworfen und die Ölphase nochmals mit 300 g Wasser gewaschen. Man erhält 2 298 g Ölphase, die im Vakuum bei 133 mbar destilliert wird. Man erhält als Hauptfraktion, 511 g, DCPB mit 97 %iger Reinheit. Diese Reinheit reicht aus zur Herstellung des Amids. Die Ausbeute beträgt 75 %, bezogen auf Einsatz.

Beispiele 2 bis 5 (2. Stufe)

Man benutzt die in 1 b beschriebene Apparatur und setzt die hier angegebenen Einsatzstoffmengen ein. Statt bei 136 °C CMPB zuzugeben, wird auf 60, 80, 100 und 120 °C abgekühlt und dann CMPB zugegeben. Hierbei ergeben sich folgende Zusammenhänge zwischen Reaktionstemperatur (RT), Reaktionszeit (h) und Ausbeute:

| RT in °C | Zeit in h | Ausbeute in % |
|---|---|---|
| 60 | 6 | 52 |
| 80 | 3 | 61 |
| 100 | 2 | 72 |
| 120 | 1 | 79 |

Die Reaktionszeit darf bei hohen Temperaturen nur kurz sein, weil sonst, wie oben erwähnt, vermehrt Nebenprodukte entstehen.

**Patentansprüche**

**1.** Verfahren zur Herstellung von 2,2-Dimethylcyclopropancarbonsäurebutylester durch Umsetzung von 5,5-Dimethylbutyrolacton mit Alkohol und gasförmigem Chlorwasserstoff zum 4-Chlor-4-methylpentansäurebutylester und dessen Cyclisierung mit einem Alkalialkoholat zum 2,2-Dimethylcyclopropancarbonsäurebutylester,
dadurch gekennzeichnet,
daß man als Alkoholkomponente Butanole in beiden Stufen verwendet und zunächst in einer ersten Stufe 5,5-Dimethylbutyrolacton und Butanol im Molverhältnis 1 : 1 bis 1 : 5 und Chlorwasserstoff in Abwesenheit eines Katalysators bei einer Temperatur von 40 bis 90 °C umsetzt, über eine Kolonne Butanol und Wasser abdestilliert und ohne Reinigung den rohen 4-Chlor-4-methylpentansäurebutylester in die 2. Stufe einsetzt, bei der dieser Ester mit einer Lösung eines Natriumbutylats in dem entsprechenden Butanol bei 80 bis 160 °C und einem Molverhältnis von Alkoholat zur Chlorverbindung von 1 : 1 bis 3 : 1 cyclisiert.

**2.** Verfahren nach Anspruch 1,
dadurch gekennzeichnet,
daß man in der ersten Stufe 5,5-Dimethylbutyrolacton und Butanol im Molverhältnis 1 : 2 bis 1 : 3 einsetzt.

**3.** Verfahren nach den Ansprüchen 1 und 2,
dadurch gekennzeichnet,
daß man die Umsetzung bei 50 bis 70 °C durchführt.

**4.** Verfahren nach Anspruch 1,
dadurch gekennzeichnet,
daß man in der zweiten Stufe bei einem Molverhältnis Alkoholat zu 4-Chlor-4-methylpentansäurebutylester von 1,8 : 1 bis 1,4 : 1 cyclisiert.

**5.** Verfahren nach den Ansprüchen 1 und 4,
dadurch gekennzeichnet,
daß man in der zweiten Stufe bei einer Temperatur von 140 bis 110 °C cyclisiert.

**6.** Verwendung des 2,2-Dimethylcyclopropancarbonsäurebutylesters nach Anspruch 1 zur Herstellung des entsprechenden Amids.

Europäisches Patentamt

# EUROPÄISCHER RECHERCHENBERICHT

Nummer der Anmeldung

EP 90 12 4139

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 220 412 (HÜLS)<br>* Seite 1, Zeile 1 - Seite 2, Zeile 29; Ansprüche * *<br>– – – | 1-6 | C 07 C 69/74<br>C 07 C 69/63<br>C 07 C 67/30<br>C 07 C 233/58 |
| D,Y | BULLETIN DE LA SOCIETE CHIMIQUE DE FRANCE. 1961, PARIS FR Seiten 1857 - 1860; M. JULIA ET AL: 'NOUVELLE SYNTHESE DE L a ACIDE TRANS-DIHYDROCHRYSANTHEMIQUE'<br>* Seite 1858, Spalte 2, Absatz 3 * *<br>– – – | 1-6 | |
| D,A | SYNTHESIS. Nr. 11, November 1982, STUTTGART DE Seiten 955 - 956; R. LANTZSCH: 'PHASENTRANSFER-KATALYSIERTE SYNTHESE VON CYCLOPROPANCARBONSÄURE-ESTERN'<br>* das ganze Dokument * *<br>– – – | 1-6 | |
| A | EP-A-0 205 403 (CIBA-GEIGY)<br>* Ansprüche * *<br>– – – – – | 1-6 | |

| RECHERCHIERTE SACHGEBIETE (Int. Cl.5) |
|---|
| C 07 C |

Der vorliegende Recherchenbericht wurde für alle Patentansprüche erstellt

| Recherchenort | Abschlußdatum der Recherche | Prüfer |
|---|---|---|
| Den Haag | 19 November 91 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument